# EUROPEAN PATENT APPLICATION

(11) **EP 4 617 271 A1**
(43) Date of publication of application: **17.09.2025**
(21) Application number: 24382287.1
(22) Date of filing: 15.03.2024
(51) Int. Cl.: C07D 401/14, A61P 35/00, A61K 31/4439

(54) **PROTEOLYTIC CHIMERAS FOR THE TREATMENT OF A CANCER RELATED TO CERT**

(71) Applicant: Consejo Superior De Investigaciones Científicas - CSIC, 28006 Madrid (ES); Universidad de Barcelona, 08028 Barcelona (ES)
(72) Inventor: Delgado Cirilo, Antonio, Barcelona (ES); Casas Brugulat, Josefina, Barcelona (ES); Bujons Vilas, Jordi, Barcelona (ES); Fabriàs Domingo, Gemma, Barcelona (ES); Crosas Navarro, Bernat, Barcelona (ES); Abad Saiz, Jose Luis, 08034 Barcelona (ES)
(74) Representative: Pons IP

(57) **Abstract**

USP14 inhibitors of formula **I**, wherein the meanings for the various substituents are as disclosed in the description, for use as a medicament and, particularly for the treatment of a cancer related to CERT.

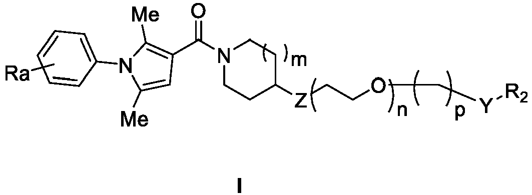

## Description

The invention relates to a USP14 inhibitor of formula I or a pharmaceutical salt thereof. The invention further relates to the compound of formula I for use as a medicament and, particularly for the treatment of a cancer related to CERT.

### BACKGROUND ART

Proteolytic targeting chimeras (Protacs) interact with an E3 ligase and a protein target, forming a ternary interaction that induces target ubiquitylation and proteasomal degradation. Proteolytic destruction of targets is a powerful strategy of inactivation of key players in human diseases, offering mechanistic advantages as compared to classical inhibition. See Pettersson and Crews (Drug Discov Today Technol. 2019; 31 :15-27), Li and Crews (Chem Soc Rev. 2022;51(12):5214-5236) and Liu and Ciulli (ACS Cent Sci. 2023;9(7):1269-1284). Initial and current efforts in Protac development have been mainly focused on Cereblon (CRBN) or Von Hippel-Lindau (VHL) E3 ligases, including chimeras derived from thalidomide and hydroxy-proline, respectively, that have generated multiple compounds with drug profile. See Pettersson and Crews (Drug Discov Today Technol. 2019; 31:15-27), Li and Crews (Chem Soc Rev. 2022;51(12):5214-5236) and Liu and Ciulli (ACS Cent Sci. 2023;9(7):1269-1284). As an example of that, the estrogen receptor degrader ARV-471, based on CRBN targeting, is currently being administered in advanced clinical trials in patients with ER+/Her2- metastatic breast cancer.

Despite the high development of the proteolytic chimera strategy, several aspects, such as the reduced number of E3-ligases, the small molecule ligands adaptable for chimera building and the poor solubility of the ligands, limit E3-based Protac implementation. An additional constraint is that ubiquitylation of the protein target is a requisite for a Protac to be efficient. Thus, proper levels and activation state of the ligase (in target tissues), productive engagement of the substrate, and accessibility of lysine residues on the target for ubiquitin modification are required for Protacs to be active. These intrinsic limitations make efforts in developing E3-based Protacs unfruitful in multiple cases. Beyond the use of E3-based degraders, different modes of targeting and degradation are under development. Hence, diverse technologies including ligands that induce degradation by means of hydrophobic moieties (Hytacs), autophagy inducers (Autacs and Attecs), plasma membrane endocytic internalizers (Lytacs) have been defined. See Pettersson and Crews (Drug Discov Today Technol. 2019; 31 :15-27), Li and Crews (Chem Soc Rev. 2022;51(12):5214-5236) and Liu and Ciulli (ACS Cent Sci. 2023;9(7):1269-1284).

The 26S proteasome holoenzyme is conformed of the core particle (CP or 20S), a cylindrical proteolytic complex, and the regulatory particle (19S or RPs), consisting of the lid and base subcomplexes (see Lander et al. Nature. 2012;482(7384):186-191). In the base, the hexameric motor ring of the AAA family of adenosine triphosphatase (ATPase) regulates substrate unfolding and translocation. The 19S also integrates substrate receptors and coordinates the activity of proteasomal deubiquitinating enzymes.

Notably, the tightly regulated process of protein degradation by the proteasome can be viewed as a sequence of events in which ubiquitin tagging of substrates does not play a strictly proteolytic role (see Bard et al., Cell. 2019;177(2):286-298). Despite the fact that ubiquitination plays a pivotal role in substrate selection, recruitment, localization and may assist the degradation of partially proteolyzed proteins retained in the 26S, the event that initiates the process of degradation by the proteasome is the engagement of a flexible fragment of the substrate by the ATPase motor, and all the consequent downstream steps are ubiquitin-independent for proteins that readily unfold (see Bard et al., Cell. 2019;177(2):286-298). Indeed, there is evidence of ubiquitin-independent degradation of endogenous and engineered protein substrates by the 26S (see Janse et al., J Biol Chem. 2004;279(20):21415-21420). In these cases, the triggers that promote degradation are adaptor factors, affinity surfaces or small molecules involved in recruitment and localization of specific substrates.

From this point of view, the proteasome contains multiple subunits and regions which may act as docking sites in a chimera-based TPD (Targeted Protein Degradation) approach. Within the 19S, the base subcomplex exposes part of the hexameric AAA-ATPases ring and pore, and subunit Rpt2 has been used as an adaptable surface for ubiquitin-independent substrate recruitment. Moreover, Rpn1, Rpn2, Rpn10, Rpn13 and Usp14, in the base, and Rpn11, in the lid, are subunits directly involved in substrate recruitment and processing, exhibiting a potential for ligand design, being the definition of amenable docking sites in their surfaces the cornerstone for such application.

The degradation of ubiquitylated proteins by the 26S is a tightly regulated process. A first regulated step is the removal of ubiquitin chains from substrates by proteasomal deubiquitylating enzymes RPN11, USP14 and UCH37. Among them, USP14 confers the ability to edit and reject substrates by a highly sophisticated allostery. During the process of substrate degradation, USP14 induces 26S state transitions and exerts the control at the steps of ubiquitin recognition, substrate translocation initiation and ubiquitin chain recycling: 1) Dynamic USP14-ATPase interactions decouple the ATPase activity from RPN11-catalysed deubiquitylation allowing USP14 to outpace RPN11 and allosterically compete with RPN11. 2) USP14 activates the ATPase motor by means of interactions with Rpt1 (despite that, USP14 deubiquitinating activity still outpaces ATPases). 3) USP14-26S proteasomes, in exiting conformational states, dissociate trimmed ubiquitin slower, causing a delay in substrate reloading (see Zhang et al., Nature. 2022;605(7910):567-574). As a consequence of this intricate triple checkpoint, USP14 was initially identified as a delayer of proteasomal activity. Furthermore, chemical inhibition of USP14 with specific USP14 inhibitors **(IU1** compounds) (see Lee et al., Nature. 2010;467(7312):179-184), due to the reactivation of RPN11 deubiquitylating roles and faster ubiquitin recycling, has an activator effect of proteasomal activity.

Therefore, IU1 compounds appear a suitable small-molecule ligands to be used in a chimera approach for targeting proteins directly to the proteasome for their degradation.

The ceramide transport protein (CERT or COL4A3BP) is a lipid transfer protein that mediates the transport of ceramide from the endoplasmic reticulum (ER) to the Golgi apparatus for sphingomyelin synthesis, where ceramide acts as the precursor of more complex sphingolipids. Owing to the ubiquity of sphingolipids in membranes and their key role of ceramide as inducer of programmed cell death, strategies that lead to accumulation of intracellular ceramide have proven successful at driving cell death in proliferative diseases such as cancers (Hannun and Obeid, 2018). Interestingly, CERT inhibition, which blocks ceramide transport to Golgi, prevents the consequent synthesis of sphingomyelin and causes the increase of ceramide intracellular levels, has been defined as a potential enhancer of anti-cancer treatments, such as chemotherapy (see Swanton et al, Cancer Cell. 2007;11(6):498-512). Furthermore, the linkage of certain constitutively active *CERT* mutants with Intellectual-disability-associated disorders (see Muramaki et al., PLoS One. 2020;15(12):e0243980.) expands the interest of CERT inhibition to additional therapeutic applications.

Therefore, it would be desirable to develop chimeras that interact with the proteasomal-associated factor Usp14 and with CERT. These novel chimeras would define mechanistically a new type of Protac targeting directly the 26S proteasome in a E3-independent manner, offering the possibility to rapidly deplete CERT from human cells. Efficient degradation of CERT would provide a beneficial effect in human breast cancer cells, which is initially tested in breast cancer cell lines. Additionally, as observed in the current invention, chimeras targeting CERT produce a sensitizing effect in cells exhibiting resistance to HER2-targeted therapies, defining a therapeutic potential of the new compounds.

### SUMMARY OF THE INVENTION

A first aspect of the present invention related to a compound of formula I: 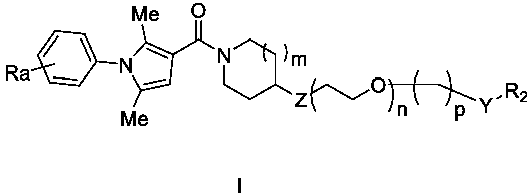
or a pharmaceutical salt thereof, wherein
Rₐ represents -H, -F, -Cl, -Br, -I, -CN, -(C₁-C₄)-alkyl, OR_{b}, -NHR_{b}R_{c}, or NO₂;
-m is an integer number between 0 and 1;
Z represents -CH₂-, -CH₂OCONR_{d}-, -CH₂NRₑCONR_{d}-, -CH₂NRₑCO- or -CH₂CONR_{d}-;
Each R_{b}, R_{c}, R_{d} and Rₑ independently represents -H- or -(C₁-C₄)-alkyl;
n is an integer number between 0 and 10;
p is an integer number between 0 and 3;
Y represents (CH₂)r or a group of formula **II**: 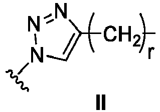
r is an integer number between 6 and 12;
R₂ represents a group of formula **III** or **IV:** 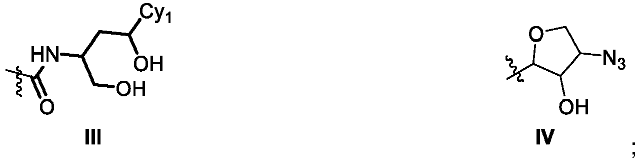 and
Cy, represents an aromatic carbocycle group from 5 to 10 carbon atoms.

In another embodiment the invention relates to the compound of formula **I** as defined above, wherein Rₐ is selected from -F, -Cl and -Br, and preferably wherein Rₐ is -F.

In another embodiment the invention relates to the compound of formula **I** as defined above, wherein m is 1.

In another embodiment the invention relates to the compound of formula **I** as defined above, wherein Z is -CH₂ or -CH₂OCONR_{d}-.

In another embodiment the invention relates to the compound of formula **I** as defined above, wherein n is an integer number from 0 to 5, and preferably wherein n is 0, 1, 2, 3 or 5.

In another embodiment the invention relates to the compound of formula **I** as defined above, wherein p is an integer number from 0 to 2, and preferably wherein p is 2.

In another embodiment the invention relates to the compound of formula **I** as defined above, wherein r is 8.

In another embodiment the invention relates to the compound of formula **I** as defined above, wherein R₂ is a group of formula **III.**

In another embodiment the invention relates to the compound of formula **I** as defined above, which is selected from:

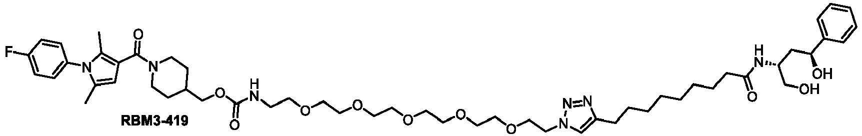

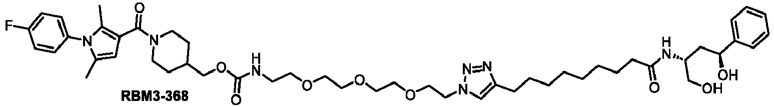

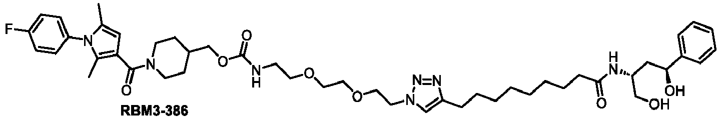

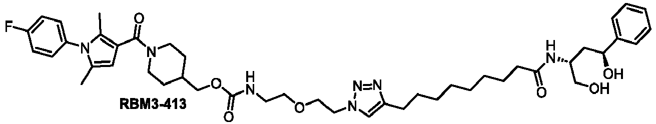

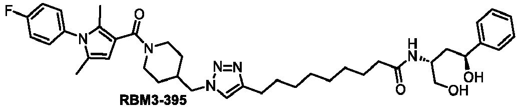

In another embodiment the invention relates to the compound of formula **I** as defined above, which is selected from: 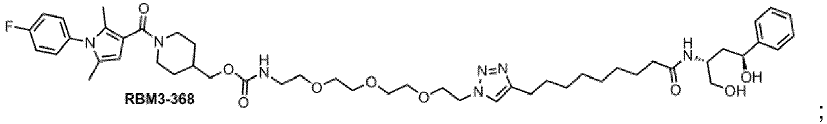 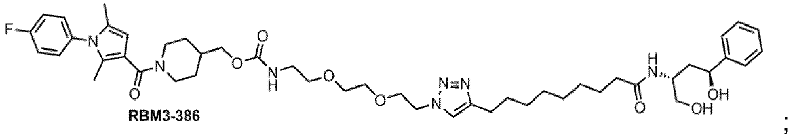 and

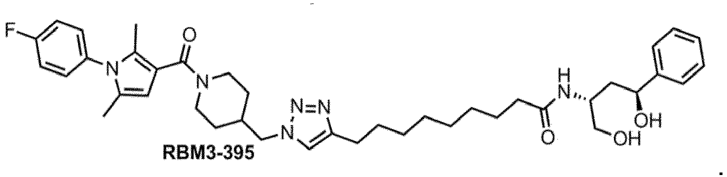

Another aspect of this invention relates to a pharmaceutical composition, which comprises a compound of formula **I** or a pharmaceutically acceptable salt thereof and one or more pharmaceutically acceptable excipients.

Another aspect of the invention relates to the compound of formula **I** or a pharmaceutical salt thereof for use as a medicament.

Another aspect of the invention relates to the compound of formula **I** or a pharmaceutical salt thereof for use in the treatment of a cancer related to CERT.

In another embodiment, the invention relates to the compound of formula **I** or a pharmaceutical salt thereof for the use defined above, wherein the cancer related to CERT is selected from lung, breast, colon, liver and ovary.

Another aspect of the present invention relates to the use of a compound of formula **I** or a pharmaceutically acceptable salt thereof for the manufacture of a medicament.

Another aspect of the present invention relates to the use of a compound of formula **I** or a pharmaceutically acceptable salt thereof for the manufacture of a medicament for the treatment of a cancer related to CERT, and preferably wherein the cancer related to CERT is selected from lung, breast, colon, and ovary.

Another aspect of the present invention relates to a method of treating a disease in a subject in need thereof, especially a human being, which comprises administering to said subject an effective amount of a compound of formula **I** or a pharmaceutically acceptable salt thereof.

Another aspect of the present invention relates to a method of treating a cancer related to CERT, preferably wherein the cancer related to CERT is selected from lung, breast, colon, and ovary, in a subject in need thereof, especially a human being, which comprises administering to said subject an effective amount of a compound of formula **I** or a pharmaceutically acceptable salt thereof.

The present invention also relates to a pharmaceutical composition that comprises a compound of the present invention (or a pharmaceutically acceptable salt or solvate thereof) and one or more pharmaceutically acceptable excipients. The excipients must be "acceptable" in the sense of being compatible with the other ingredients of the composition and not deleterious to the recipients thereof.

The compounds of the present invention can be administered in the form of any pharmaceutical formulation, the nature of which, as it is well known, will depend upon the nature of the active compound and its route of administration. Any route of administration may be used, for example oral, parenteral, nasal, ocular, rectal and topical administration.

Solid compositions for oral administration include tablets, granulates and capsules. In any case the manufacturing method is based on a simple mixture, dry granulation or wet granulation of the active compound with excipients. These excipients can be, for example, diluents such as lactose, microcrystalline cellulose, mannitol or calcium hydrogenphosphate; binding agents such as for example starch, gelatin or povidone; disintegrants such as sodium carboxymethyl starch or sodium croscarmellose; and lubricating agents such as for example magnesium stearate, stearic acid or talc. Tablets can be additionally coated with suitable excipients by using known techniques with the purpose of delaying their disintegration and absorption in the gastrointestinal tract and thereby provide a sustained action over a longer period, or simply to improve their organoleptic properties or their stability. The active compound can also be incorporated by coating onto inert pellets using natural or synthetic film-coating agents. Soft gelatin capsules are also possible, in which the active compound is mixed with water or an oily medium, for example coconut oil, mineral oil or olive oil. Powders and granulates for the preparation of oral suspensions by the addition of water can be obtained by mixing the active compound with dispersing or wetting agents; suspending agents and preservatives. Other excipients can also be added, for example sweetening, flavouring and colouring agents.

Liquid forms for oral administration include emulsions, solutions, suspensions, syrups and elixirs containing commonly-used inert diluents, such as purified water, ethanol, sorbitol, glycerol, polyethylene glycols (macrogols) and propylene glycol. Said compositions can also contain coadjuvants such as wetting, suspending, sweetening, flavouring agents, preservatives and buffers.

Injectable preparations, according to the present invention, for parenteral administration, comprise sterile solutions, suspensions or emulsions, in an aqueous or non-aqueous solvent such as propylene glycol, polyethylene glycol or vegetable oils. These compositions can also contain coadjuvants, such as wetting, emulsifying, dispersing agents and preservatives. They may be sterilized by any known method or prepared as sterile solid compositions, which will be dissolved in water or any other sterile injectable medium immediately before use. It is also possible to start from sterile materials and keep them under these conditions throughout all the manufacturing process.

For the rectal administration, the active compound can be preferably formulated as a suppository on an oily base, such as for example vegetable oils or solid semisynthetic glycerides, or on a hydrophilic base such as polyethylene glycols (macrogol).

The compounds of the invention can also be formulated for their topical application for the treatment of pathologies occurring in zones or organs accessible through this route, such as eyes, skin and the intestinal tract. Formulations include creams, lotions, gels, powders, solutions and patches wherein the compound is dispersed or dissolved in suitable excipients.

For the nasal administration or for inhalation, the compound can be formulated as an aerosol, and it can be conveniently released using suitable propellants.

The dosage and frequency of doses will depend upon the nature and severity of the disease to be treated, the age, the general condition and body weight of the patient, as well as the particular compound administered and the route of administration, among other factors. A representative example of a suitable dosage range is from about 0.01 mg/Kg to about 100 mg/Kg per day, which can be administered as single or divided doses.

There is no limitation on the type of salt that can be used, provided that these are pharmaceutically acceptable when used for therapeutic purposes. The term pharmaceutically acceptable salt refers to those salts which are, according to medical judgment, suitable for use in contact with the tissues of humans and other mammals without undue toxicity, irritation, allergic response and the like. Pharmaceutically acceptable salts are well known in the art.

The salts of a compound of formula **I** can be obtained during the final isolation and purification of the compounds of the invention or can be prepared by treating a compound of formula **I** with a sufficient amount of the desired acid or base to give the salt in a conventional manner. The salts of the compounds of formula **I** can be converted into other salts of the compounds of formula **I** by ion exchange using ionic exchange resins.

The compounds of formula **I** and their salts may differ in some physical properties, but they are equivalent for the purposes of the present invention. All salts of the compounds of formula **I** are included within the scope of the invention.

The compounds of the present invention may form complexes with solvents in which they are reacted or from which they are precipitated or crystallized. These complexes are known as solvates. As used herein, the term solvate refers to a complex of variable stoichiometry formed by a solute (a compound of formula **I** or a salt thereof) and a solvent. Examples of solvents include pharmaceutically acceptable solvents such as water, ethanol and the like. A complex with water is known as a hydrate. Solvates of compounds of the invention (or salts thereof), including hydrates, are included within the scope of the invention.

The compounds of formula **I** may exist in different physical forms, i.e. amorphous and crystalline forms. Moreover, the compounds of the invention may have the ability to crystallize in more than one form, a characteristic which is known as polymorphism. Polymorphs can be distinguished by various physical properties well known in the art such as X-ray diffraction pattern, melting point or solubility. All physical forms of the compounds of formula **I**, including all polymorphic forms ("polymorphs") thereof, are included within the scope of the invention.

Some of the compounds of the present invention may exist as several diastereoisomers and/or several optical isomers. Diastereoisomers can be separated by conventional techniques such as chromatography or fractional crystallization. Optical isomers can be resolved by conventional techniques of optical resolution to give optically pure isomers. This resolution can be carried out on any chiral synthetic intermediate or on products of formula **I**. Optically pure isomers can also be individually obtained using enantiospecific synthesis. The present invention covers all individual isomers as well as mixtures thereof (for example racemic mixtures or mixtures of diastereomers), whether obtained by synthesis or by physically mixing them.

In the above definitions, the term C₁-C₄ alkyl, as a group or part of a group, means a straight or branched alkyl chain which contains from 1 to 4 carbon atoms and includes the groups methyl, ethyl, propyl, isopropyl, butyl, isobutyl, sec-butyl and tert-butyl.

When in the definitions used throughout the present specification for cyclic groups the examples given refer to a radical of a ring in general terms, for example phenyl, all possible positions of attachment are included, unless any limitation is mentioned in the definition of the corresponding group. Thus, for example, in the definitions for Cy₁, which do not include any limitation with regard to the position of attachment, the term phenyl includes 2-phenyl, 3-phenyl and 4-phenyl.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration and are not intended to be limiting of the present invention.

### DESCRIPTION OF THE DRAWINGS

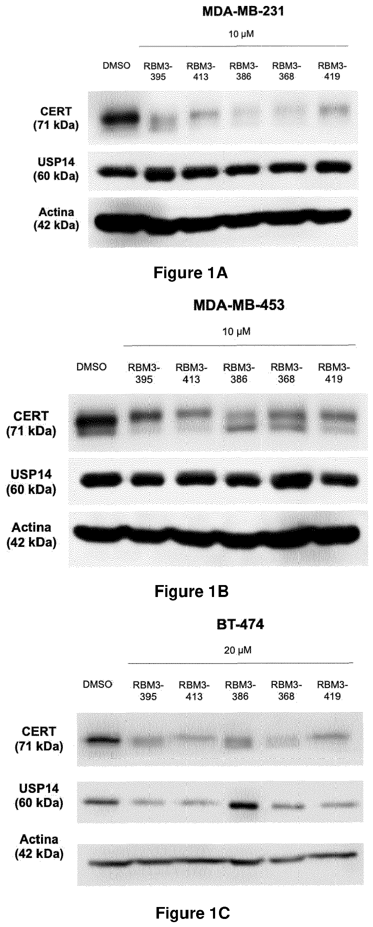

Figure 1. Degradation of CERT in breast cancer cell lines: MDA-MB-231 (A), MDA-MB-453/WT (B) and BT-474/WT (C) with PROTACs RBM3-395, RBM3-413, RBM3-386, RBM3-368 and RBM3-419. Cells were treated with compounds or vehicle (DMSO) for 24 hours. In MDA-MB-231 and MDA-MB-453/WT cells compounds were administered at 10 µM and in BT-474/WT, at 20 µM. Cells were analyzed by SDS-PAGE followed by immunoblotting using antibodies against CERT, USP14 and Actin. The decreased signal of CERT in treated samples with respect to DMSO samples indicate that treatments have reduced the levels of CERT in cells.

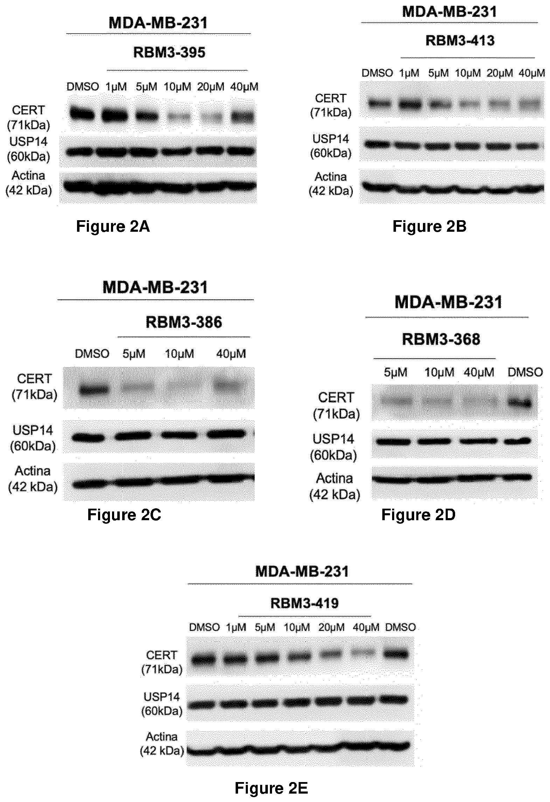

Figure 2. Degradation of CERT in MDA-MB-231 cells with chimeras RBM3-395 (A), RBM3-413 (B), RBM3-386 (C), RBM3-368 (D), RBM3-419 (E). Treatments for 24 hours at different chimera concentration (1, 5, 10, 20 and 40 µM) are shown. Cells were analyzed by SDS-PAGE followed by immunoblotting using antibodies against CERT, USP14 and Actin. The decreased signal of CERT in treated samples with respect to DMSO samples indicate that treatments have reduced the levels of CERT in cells.

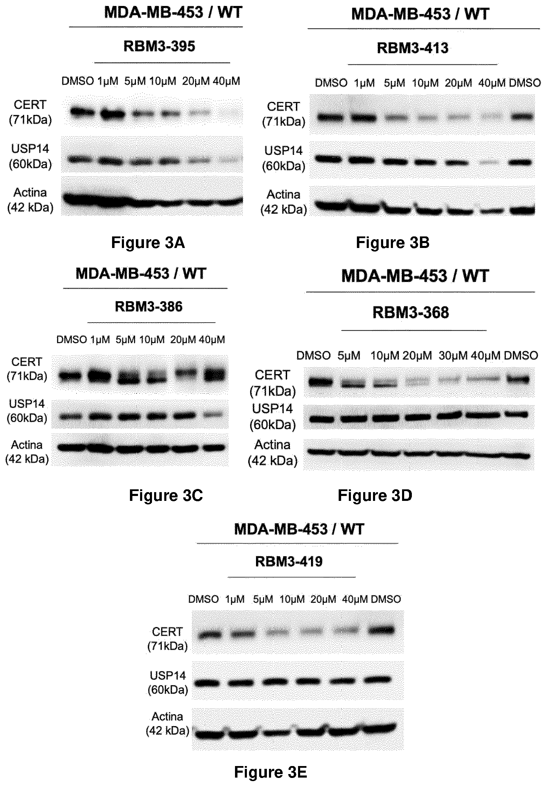

Figure 3. Degradation of CERT in MDA-MB-453 cells. Panels show treatments as follows: MDA-MB-453/WT cells with chimeras RBM3-395 (A), RBM3-413 (B), RBM3-386 (C), RBM3-368 (D), RBM3-419 (E). Treatments for 24 hours at different chimera concentration (1, 5, 10, 20, 30 and 40 µM) are shown. Cells were analyzed by SDS-PAGE followed by immunoblotting using antibodies against CERT, USP14 and Actin. The decreased signal of CERT in treated samples with respect to DMSO samples indicate that treatments have reduced the levels of CERT in cells.

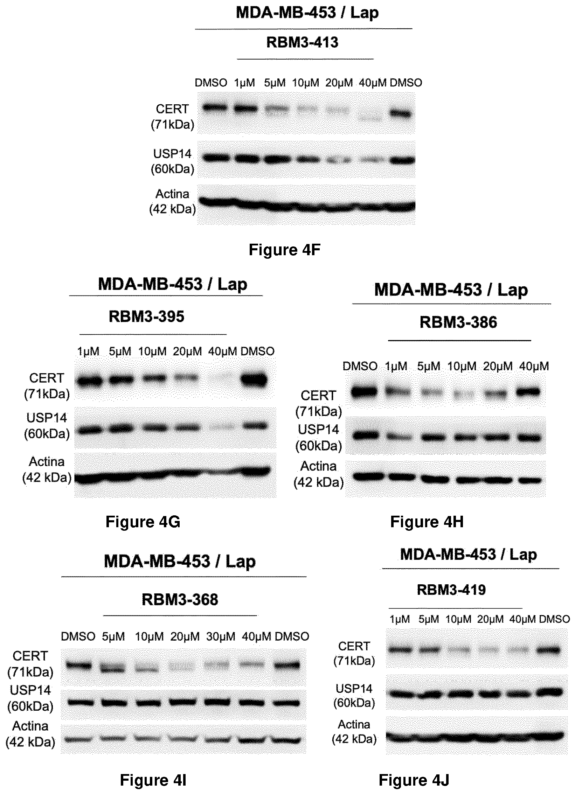

Figure 4. Degradation of CERT in MDA-MB-453 cells. Panels show treatments as follows: MDA-MB-453 Lapatinib resistant cells (MDA-MB-453/Lap) with chimeras RBM3-395 (F), RBM3-413 (G), RBM3-386 (H), RBM3-368 (I), RBM3-419 (J). Treatments for 24 hours at different chimera concentration (1, 5, 10, 20, 30 and 40 µM) are shown. Cells were analyzed by SDS-PAGE followed by immunoblotting using antibodies against CERT, USP14 and Actin. The decreased signal of CERT in treated samples with respect to DMSO samples indicate that treatments have reduced the levels of CERT in cells.

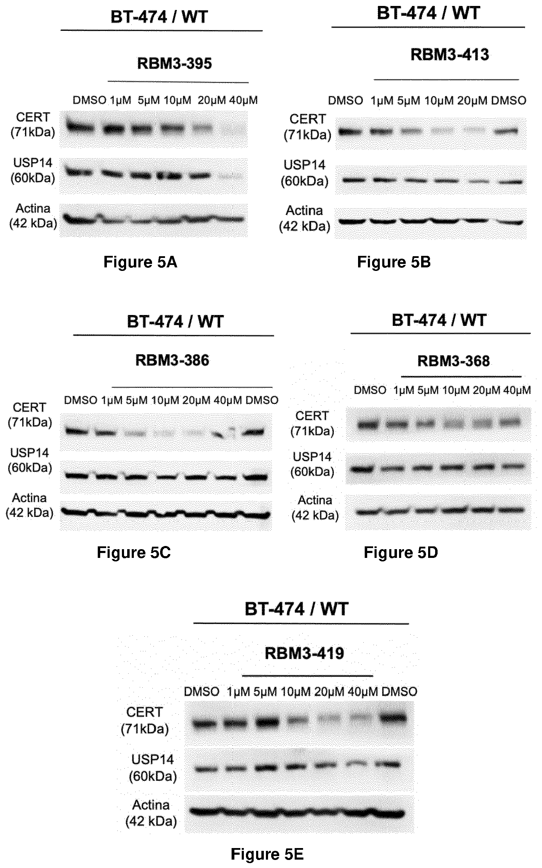

Figure 5. Degradation of CERT in BT-474 cells. Panels show treatments as follows: BT-474/WT cells with chimeras RBM3-395 (A), RBM3-413 (B), RBM3-386 (C), RBM3-368 (D), RBM3-419 (E). BT-474 Treatments for 24 hours at different chimera concentration (1, 5, 10, 20, 30 and 40 µM) are shown. Cells were analyzed by SDS-PAGE followed by immunoblotting using antibodies against CERT, USP14 and Actin. The decreased signal of CERT in treated samples with respect to DMSO samples indicate that treatments have reduced the levels of CERT in cells.

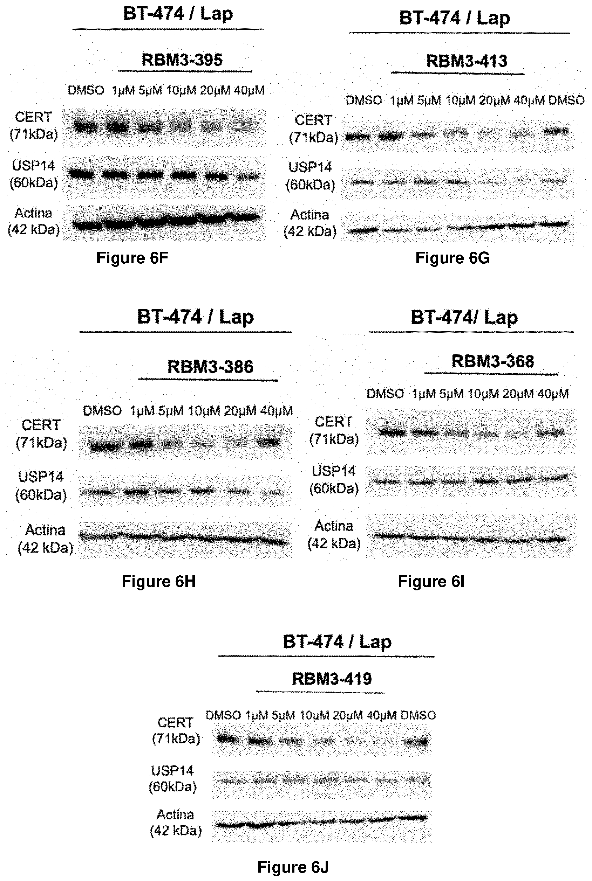

Figure 6. Degradation of CERT in BT-474 cells. Panels show treatments as follows: BT-474 Lapatinib resistant cells (BT-474/Lap) with chimeras RBM3-395 (F), RBM3-413 (G), RBM3-386 (H), RBM3-368 (I), RBM3-419 (J). Treatments for 24 hours at different chimera concentration (1, 5, 10, 20, 30 and 40 µM) are shown. Cells were analyzed by SDS-PAGE followed by immunoblotting using antibodies against CERT, USP14 and Actin. The decreased signal of CERT in treated samples with respect to DMSO samples indicate that treatments have reduced the levels of CERT in cells.

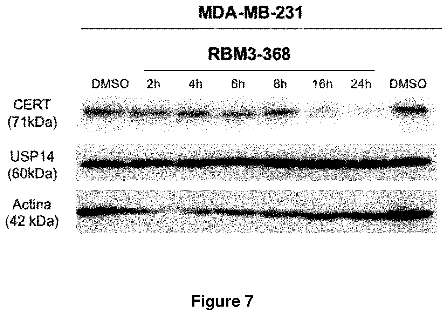

Figure 7. Time-course degradation of CERT in MDA-MB-231 cells with chimera RBM3-368. Treatments were performed at 10 µM for 2, 4, 6, 8, 16 and 24h. Cells were analyzed by SDS-PAGE followed by immunoblotting using antibodies against CERT, USP14 and Actin. The decreased signal of CERT in treated samples with respect to DMSO samples indicate that treatments have reduced the levels of CERT in cells.

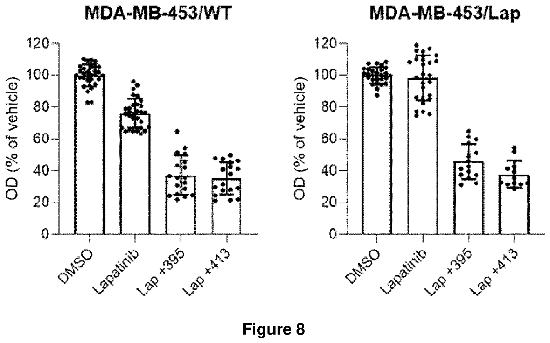

Figure 8. Viability of wild type (MDA-MB-453/WT) and lapatinib (Lap) resistant (MDA-MB-453/Lap) MDA-MB-453 cells treated with lapatinib (10 µM) in the absence or the presence of the Protacs RBM3-395 or RBM3-413 (10 µM) for 24 h. Data are the mean +/- SD of 4-5 experiments with triplicates.

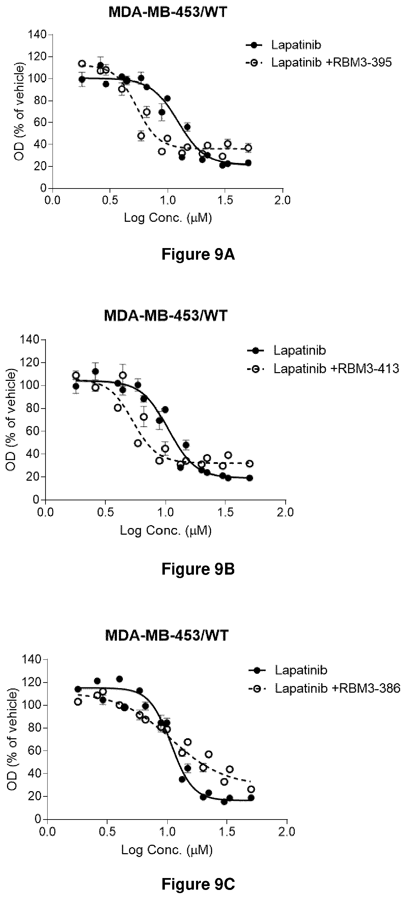

Figure 9. Viability of wild type (MDA-MB-453/WT) and lapatinib (Lap) resistant (MDA-MB-453/Lap) MDA-MB-453 cells treated with different concentrations (3-50 µM) of lapatinib in the absence or the presence of the Protacs (10 µM) for 24 h. Graphs show logarithmic representation of Lapatinib concentration versus viability of: A. MDA-MB-453/WT cells with RBM3-395. B. MDA-MB-453/WT cells with RBM3-413. C. MDA-MB-453/WT cells with RBM3-386. Data are the mean +/- SD of 3 experiment with triplicates.

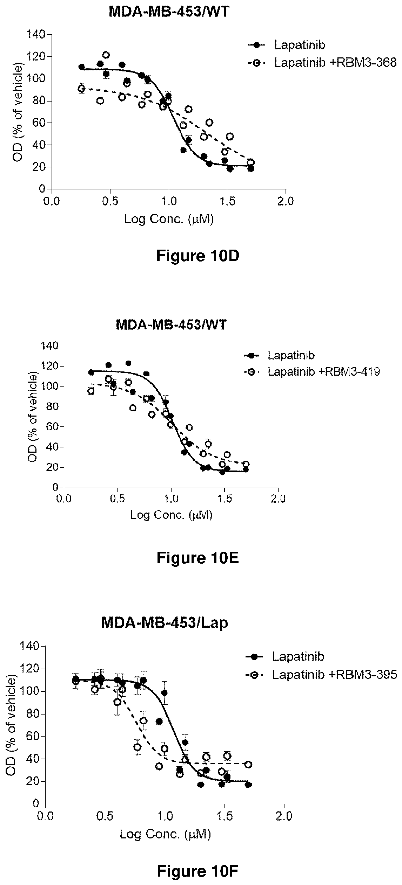

Figure 10. Viability of wild type (MDA-MB-453/WT) and lapatinib (Lap) resistant (MDA-MB-453/Lap) MDA-MB-453 cells treated with different concentrations (3-50 µM) of lapatinib in the absence or the presence of the Protacs (10 µM) for 24 h. Graphs show logarithmic representation of Lapatinib concentration versus viability of: D. MDA-MB-453/WT cells with RBM3-368. E. MDA-MB-453/WT cells with RBM3-419. F. MDA-MB-453/Lap cells with RBM3-395. Data are the mean +/- SD of 3 experiment with triplicates.

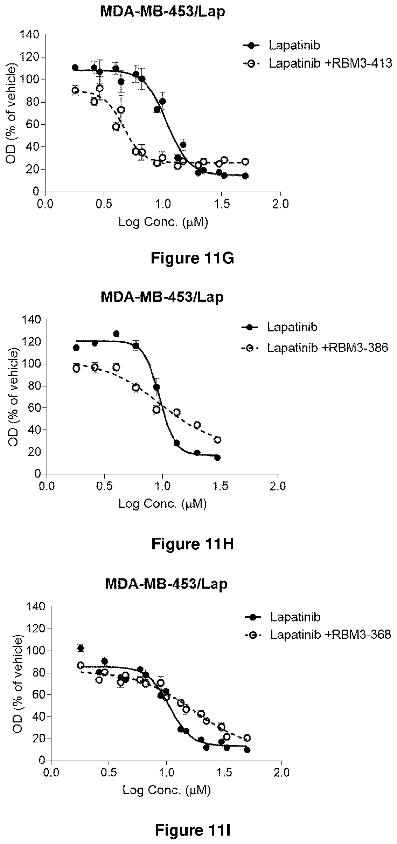

Figure 11. Viability of wild type (MDA-MB-453/WT) and lapatinib (Lap) resistant (MDA-MB-453/Lap) MDA-MB-453 cells treated with different concentrations (3-50 µM) of lapatinib in the absence or the presence of the Protacs (10 µM) for 24 h. Graphs show logarithmic representation of Lapatinib concentration versus viability of: G. MDA-MB-453/Lap cells with RBM3-413. H. MDA-MB-453/Lap cells with RBM3-386. I. MDA-MB-453/Lap cells with RBM3-368. J MDA-MB-453/Lap cells with RBM3-419.. Data are the mean +/- SD of 3 experiment with triplicates.

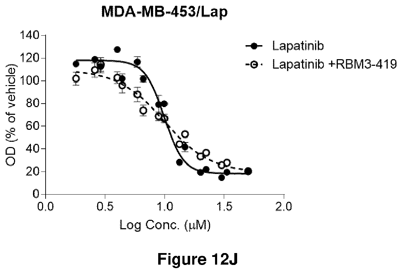

Figure 12. Viability of wild type (MDA-MB-453/WT) and lapatinib (Lap) resistant (MDA-MB-453/Lap) MDA-MB-453 cells treated with different concentrations (3-50 µM) of lapatinib in the absence or the presence of the Protacs (10 µM) for 24 h. Graphs show logarithmic representation of Lapatinib concentration versus viability of: J MDA-MB-453/Lap cells with RBM3-419.. Data are the mean +/- SD of 3 experiment with triplicates.

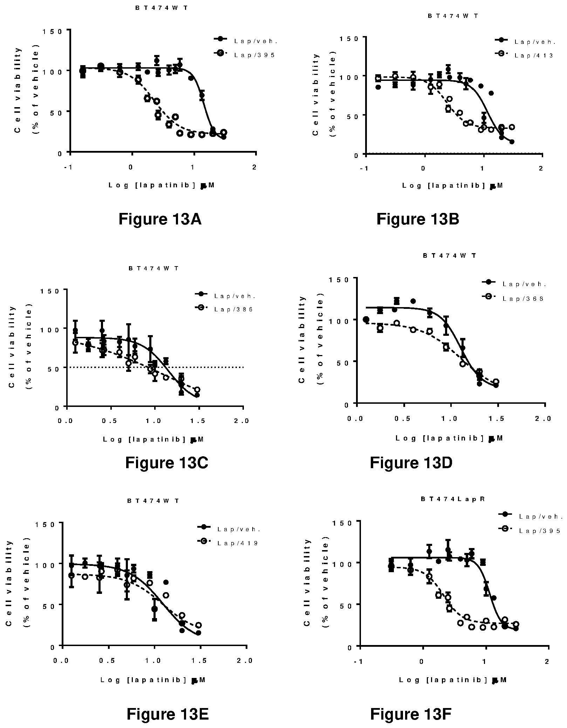

Figure 13. Viability of wild type (BT-474/WT) and lapatinib (Lap) resistant (BT-474/Lap) BT-474 cells treated with different concentrations (0.63-30 µM) of lapatinib in the absence or the presence of the Protacs (20 µM) for 24 h. Graphs show logarithmic representation of Lapatinib concentration versus viability of: A. BT474/WT cells with RBM3-395. B. BT474/WT cells with RBM3-413. C. BT474/WT cells with RBM3-386. D. BT474/WT cells with RBM3-368. E. BT474/WT cells with RBM3-419. F. BT474/Lap cells with RBM3-395. Data are the mean +/- SD of 3 to 5 experiments with triplicates.

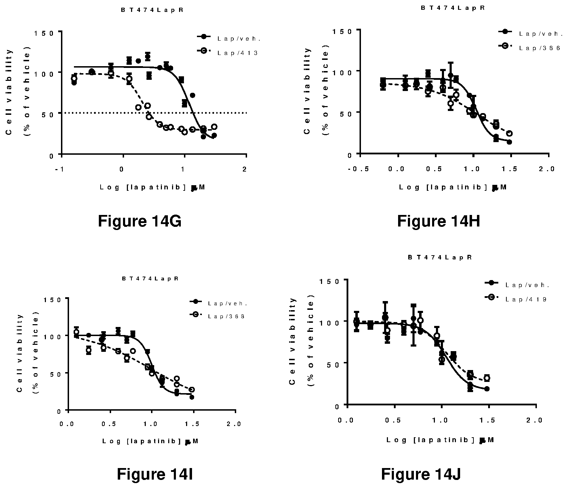

Figure 14. Viability of wild type (BT-474/WT) and lapatinib (Lap) resistant (BT-474/Lap) BT-474 cells treated with different concentrations (0.63-30 µM) of lapatinib in the absence or the presence of the Protacs (20 µM) for 24 h. Graphs show logarithmic representation of Lapatinib concentration versus viability of: G. BT474/Lap cells with RBM3-413. H. BT474/Lap cells with RBM3-386. I. BT474/Lap cells with RBM3-368. J BT474/Lap cells with RBM3-419. Data are the mean +/- SD of 3 to 5 experiments with triplicates.

### Examples

### Reagents and general methods

All chemicals were purchased from commercial sources and used as received unless otherwise noted. Dry solvents (THF, DMF and CH₂Cl₂), obtained from a PureSolv dispenser and subsequently degassed with inert gas, were used in most reactions. Synthesis grade (Hexane, EtOAc, Et₂O and CH₂Cl₂) or HPLC-grade (CH₃OH) solvents were used for extractions and purifications. Unless otherwise specified all reactions were performed under Ar inert atmosphere. Progression of the reactions was controlled by thin layer chromatography, using ALUGRAM^{®} SIL G/UV254 (Macherey-Nagel) silica gel pre-coated aluminum sheets (Layer: 0.2 mm, silica gel 60). Compounds were detected by using UV light (λ=254 nm) and a stain solution of phosphomolibdic acid (5.7% in EtOH). Compounds were purified by flash column chromatography, using silica gel (Chromatogel 60 Å, 35-75 µm) as stationary phase. Mobile phases and gradients are specified in each case in the following section. ¹H and ¹³C Nuclear Magnetic Resonance spectra were recorded on a Varian - Mercury 400 (¹H NMR at 400 MHz and ¹³C NMR at 100.6 MHz) spectrometer using CDCl₃ or CD₃OD as solvent. Chemical shifts of deuterated solvents were used as internal standards. Chemical shifts are given in parts per million (ppm). Splitting patterns have been described as singlet (s), broad singlet (br s), doublet (d), triplet (t), quartet (q), quintuplet (p), multiplet (m), apparent (app) or combinations of these descriptive names. High-resolution mass spectra were recorded Waters Aquity ultraperformance liquid chromatography system connected to a Waters LCT Premier Orthogonal Accelerated Time of Flight Mass Spectrometer (Waters,Milford, MA, USA) operated in positive electrospray ionisation mode. Samples were analyzed by Flow Injection Analysis, using CH₃CN/water (70:30) as mobile phase. Samples were analyzed using a 10 µL volume injection. M/z ratios are reported as values in atomic mass units. N3-PEG(3)-NH2 **(381)** was purchased from Biopharma PEG Scientific Inc.

### Abbreviations

ATPase, Adenosinetriphosphatase; CC50, 50% cytotoxic concentration; CERT, Ceramide transport protein; CRBN, Cereblon; DMEM, Dulbecco's Modified Eagle Medium; DMF, Dimethylformamide; DMSO, Dimethylsulfoxide; DMSO, Dimethylsulfoxide; EDC, 3-(Ethyliminomethyleneamino)-N,N-dimethylpropan-1-amine; ER, Endoplasmic reticulum; ER, Estrogen receptor; Et₂O, Diethyl ether; EtOAc, Ethyl acetate; EtOH, Ethanol; FBS, Fetal bovine serum; HER2, Human epidermal growth factor receptor-2; HOBt,·Hydroxybenzotriazole; HPLC, High performance liquid chromatography; Lap, Lapatinib; MTBE, Methyl tert-butyl ether; MTT, 3-[4,5-Dimethylthiazol-2-yl]-2,5 diphenyl tetrazolium bromide; NMR, Nuclear magnetic resonance; PBS, Phosphate buffer saline; PEG, Polyetyleneglycol; PR, Progesterone receptor; Protac, Proteolytic targeting chimera; SDS-PAGE, Sodium dodecyl sulfate polyacrylamide gel electrophoresis; TEA, Triethylamine; TPD, Targeted Protein Degradation; VHL, Von Hippel-Lindau;

### Example 1: Synthesis of the compounds of formula I

### A. Synthesis of intermediates

### Preparation of intermediate 366

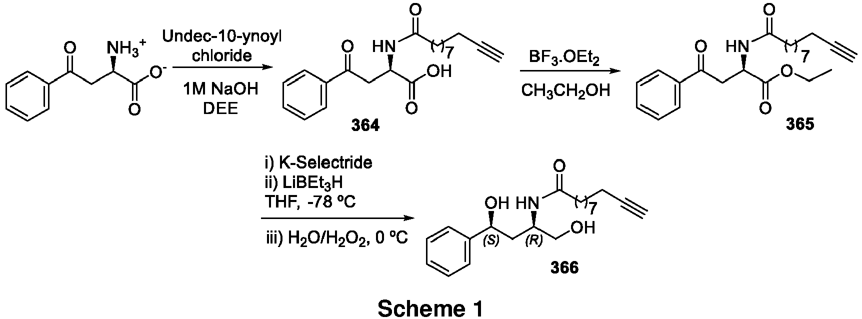

### [(R)-4-Oxo-4-phenyl-2-(undec-10-ynamido)butanoic acid] (364)

This product was prepared using Schotten-Baumann conditions. Thus, 0.8 g (3.48 mmol) of (R)-2-ammonio-4-oxo-4-phenylbutanoate was suspended in cold 2 N NaOH (6 mL) and treated with 3 equivalents of neat undec-10-ynoyl chloride freshly prepared, under vigorous stirring for 1 h.

The white cloudy suspension was acidified with 2 N aqueous HCl solution, extracted with CH₂Cl₂ (3 x 50 mL) and dried over MgSO₄. Evaporation to dryness afforded a residue which was purified by flash column chromatography on silica gel using initially a hexane/MTBE 7:3 solvent mixture (to remove undecynoic acid excess) and then a stepwise gradient solvent mixture of MeOH in CH₂Cl₂ (0-24%, 1% each) to afford 749 mg (60 % yield) of pure **364.**

¹H NMR (CDCl₃): 7.94 (app d, 2H), 7.60 (app t, 1H), 7.47 (app t, 2H), 6.76 (d, 1H), 5.02 (m, 1H), 3.79 (dd, 1H), 3.59 (dd, 1H), 2.22 (t, 2H), 2.14 (dt, 2H), 1.59 (m, 2H), 1.46 (m, 2H), 1.32 (m, 2H), 1.30-1.10 (broad, 6H). ¹³C NMR (CDCl₃): 198.3, 175.1, 174.0, 135.9, 134.0, 128.9, 128.4, 84.8. 68.2, 48.3, 40.5, 36.5, 29.2, 29.1, 29.0, 28.7, 28.5, 25.6, 18.5.

HRMS calculated for C₂₁H₂₈NO4⁺ [M+H]⁺: 358.2013; found: 358.2008.

[α]^{D} = -6.3 (c = 1.0, CHCl₃).

### [Ethyl (R)-4-oxo-4-phenyl-2-(undec-10-ynamido)butanoate] (365)

To a solution of **364** (5 mmol) in ethanol (20 mL), 1 mL of BF₃.Et₂O complex was carefully added. The reaction mixture was vigorously stirred at rt for 72h and then concentrated to dryness. The residue was taken up in H₂O (25 mL), extracted with CH₂Cl₂ (3 x 20 mL) and the organic phase washed with sat. NaHCO₃ solution (20 mL). After usual workup, the resulting extract containing the ethylester was dried under anh. MgSO₄, concentrated to a residue that was purified by flash column chromatography with silica gel (hexanes/EtOAc, stepwise gradient 2% each, 0-50%) to yield **365** (72% yield).

¹H NMR (CDCl₃): 7.94 (app d, 2H), 7.60 (app t, 1H), 7.48 (app t, 2H), 6.58 (d, 1H), 4.96 (dt, 1H), 4.21 (q, , 2H), 3.75 (dd, 1H), 3.58 (dd, 1H), 2.20 (dt, 2H), 2.15 (dt, 2H), 1.93 (t, 1H), 1.61 (m, 2H), 1.48 (m, 2H), 1.34 (m, 2H), 1.33-1.22 (broad, 6H), 1.23 (t, 3H). ¹³C NMR (CDCl₃): 198.1, 173.0, 171.3, 136.1, 133.8, 128.8, 128.2, 84.8, 68.2, 61.8, 48.3, 40.6, 36.6, 29.21, 29.15, 28.9, 28.7, 28.5, 25.6, 18.4, 14.1.

HRMS calculated for C₂₃H₃₂ NO₄⁺[M+H]⁺ : 386.2326; found: 386.2330.

[α]^{D} = -62.5 (c = 1.0, CHCl₃).

### [N-((2R,4S)-1,4-dihydroxy-4-phenylbutan-2-yl)undec-10-ynamide] (366)

To a solution of the ketoamide-ester **365** (386 mg, 1 mmol) in dry THF was added K-Selectride in THF (1.5 mL, 1 M, 1.5 mmol) at -78 °C. The mixture was stirred at the same temperature for 10 min, and then LiBEtsH in THF (2 mL, 1.7 M, 3.4 mmol) was added. The reaction mixture was allowed to warm to room temperature and further stirred for 1 h. The reaction mixture was cooled with an ice bath and quenched by dropwise careful addition of water (1 mL) and 30% aqueous H₂O₂ (1.6 mL). The mixture was extracted with CH₂Cl₂ and the extract was washed with saturated aqueous NaHCO₃. The combined aqueous layer was extracted with CH₂Cl₂, and the combined organic layer was washed with brine and dried over anhydrous MgSO₄. The solvents were evaporated, and the residue was purified by flash chromatography on silica gel using a CH₂Cl₂/CH₃OH stepwise gradient (0-6%, 0.2% each column volume) to give enantiomerically pure undec-10-ynil-HPA **366** (72% yield).

¹H NMR (CDCl₃): 7.34 and 7.33 (brs, 4H), 7.27 (m, 1H), 6.41 (d,), 4.80 (dd, 1H), 4.05 (m, 1H), 3.67 (m, 2H), 2.22-2.12 (m, 4H), 2.10-2.00 (m, 1H), 1.98-1.88 (m, 1H), 1.93 (t, 1H), 1.62 (m, 2H), 1.52 (m, 2H), 1.39 (m, 2H), 1.31 (br s, 6H).

¹³C NMR (CDCl₃): 174.5, 144.4, 128.5, 127.6, 125.7, 84.8, 71.5, 68.3, 65.0, 50.1, 40.9, 36.7, 29.2, 29.0, 28.7, 28.5, 25.8, 18.4.

HRMS calculated for C₂₁H₃₂NO₃⁺[M+H]⁺: 346.2377; found: 346.2372.

[α]^{D} = -38.0 (c = 1.0, CHCl₃).

### Preparation of intermediate 394

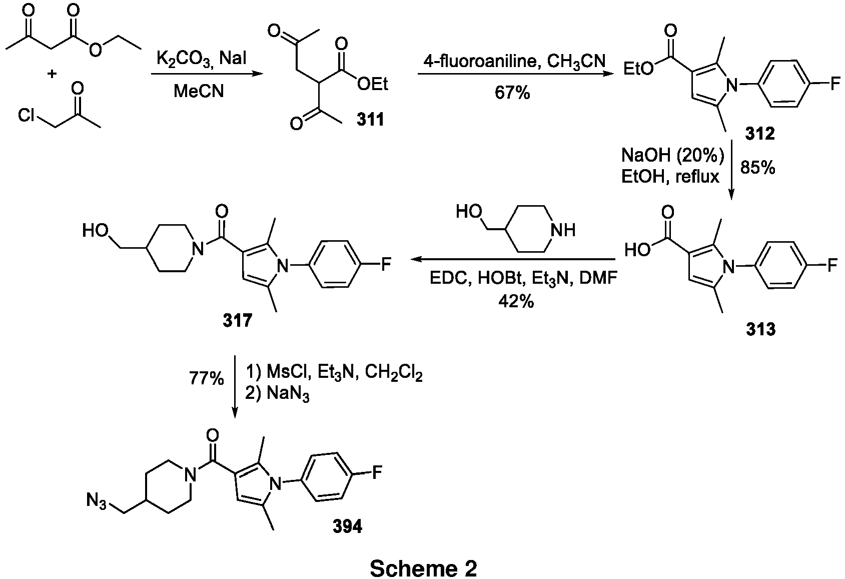

intermediates **312** and **313** were prepared following previous reported procedures and the experimental data was consistent with the literature (see Mahy et al., J. Med. Chem 2020, 63, 9483).

### [1-(4-Fluorophenyl)-2,5-dimethyl-1H-pyrrol-3-yl][4-(hydroxymethyl)piperidin-1-yl]methanone (317)

To a solution of acid **313** (1.50 g, 6.41 mmol), 4-(hydroxymethyl) piperidine (0.89 g, 7.72 mmol), HOBt·H₂O (1.28 g, 8.36 mmol), and EDC (1.97 g, 10.29 mmol) in DMF (60 mL) TEA (3.6 mL, 25.72 mmol) was added and stirred at room temperature overnight. The reaction mixture was washed with saturated NaHCO₃ and extracted with CH₂Cl₂. The combined organic extracts were washed with brine, dried over Na₂SO₄, and concentrated under reduced pressure. The crude product was dissolved in CH₃CN, filtered and purified to afford alcohol **317** (2.0 g, 94% yield) as a brown wax.

¹H NMR (400 MHz, CDCl₃) *δ* 7.23-7.14 (m, 4H), 5.97 (s, 1H), 3.66 (s, 1H), 3.56 (d, 2H), 2.09 (s, 3H), 1.98 (s, 3H), 1.86-1.82 (m, 1H), 1.81-1.79 (m, 2H), 1.73 (br, 2H), 1.33-1.18 (m, 4H). ¹³C NMR (101 MHz, CDCl₃) *δ* 168.0, 163.5, 161.0, 134.3, 130.7, 130.1, 130.0, 128.3, 116.5, 116.2, 115.1, 106.4, 70.7, 67.7, 39.3, 29.8, 12.8, 12.1.

HRMS calcd. for C₁₉H₂₄FN₂O₂⁺ ([M+H]⁺): 331.1816, found: 331.1821.

### [(4-(Azidomethyl)piperidin-1-yl)(1-(4-fluorophenyl)-2,5-dimethyl-1H-pyrrol-3-yl)methanone] (394)

Mesyl chloride (0.381 mL, 4.918 mmol) was added dropwise to a stirring solution of alcohol **317** (0.650 g, 1.967 mmol) and TEA (0.821 mL, 5.902 mmol), in anhydrous CH₂Cl₂ (19.7 mL) at 0 ºC. After the addition was complete, the mixture was allowed to warm to rt and stirred for 3 h. The reaction mixture was then poured into 0.5 M aqueous HCl (10 mL) and extracted with CH₂Cl₂ (3 x 10 mL). The combined organic layers were washed with brine, dried over anhydrous MgSO4, filtered, and evaporated to dryness.

The resulting residue was dissolved in DMF (7.6 mL) and NaN₃ (0.639 g, 9.836 mmol) was added portion-wise. After stirring overnight at 70 ºC, DMF was removed in vacuo and the residue was taken up in Et₂O (50 mL), filtered over a pad of Celite^{®}, and rinsed with Et₂O (2 x 25 mL). The combined filtrates were concentrated under vacuum to yield a crude (0.540 gthat was purified by silica gel flash column chromatography (from 0 to 10% MeOH in CH₂Cl₂) to furnish azide **394** (0.540 g, 77% yield, 2 steps).

¹H NMR (400 MHz, CDCl₃) *δ* 7.20 - 7.08 (m, 4H), 5.92 (s, 1H), 3.20 (d, 2H), 2.84 (br s, 2H), 2.04 (s, 3H), 1.94 (s, 3H), 1.77 (m, 3H), 1.22 (m, 2H). ¹³C NMR (101 MHz, CDCl₃) *δ* 167.8, 163.4, 160.9, 134.1, 130.7, 130.0, 129.9, 128.2, 116.3, 116.1, 114.9, 106.3, 57.0, 36.9, 12.6, 11.9.

HRMS calcd for C₁₉H₂₃FN₅O⁺([M+H]⁺): 356.1887, found: 356.1884.

### Preparation of N3-PEGn-NH2 linkers 396, 384 and 397

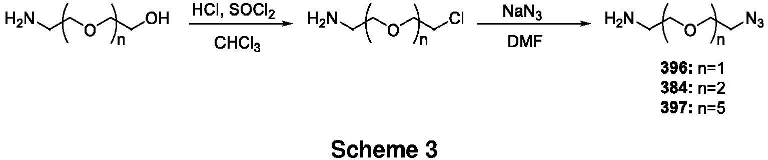

The corresponding aminoalcohol (1 equiv.) in CHCl₃ (1.6 M) was acidified with concentrated HCl, dropwise addition, (1.4 equiv.). The resulting mixture was stirred at room temperature for 6 hours and then dried under reduced pressure. The remaining residues were dissolved in CHCl₃ (1 M), followed by the slow addition (1 h) of thionyl chloride (2.7 equiv.). The reaction was allowed to stir at room temperature for 1 h and then heated to reflux for 5 h. After concentrating the mixture under reduced pressure, the resulting crude product was dissolved in 1,2-dichloroethane and washed with a saturated solution of NaHCO3. The combined organic phase was dried using anhydrous MgSO4, filtered, and evaporated to yield the expected chloramine. Without further purification the residues were solved in DMF (0.6 M) and NaN₃ (1.3 equiv.) was added. The reaction mixture was stirred at 45°C for 24 hours. After removing DMF under reduced pressure the corresponding crude was purified through flash chromatography.

### 2-(2-Azidoethoxy)ethan-1-amine (396)

¹H NMR (400 MHz, CDCl₃) *δ* 3.84 - 3.81 (m, 2H), 3.73 - 3.69 (m, 2H), 3.50 - 3.47 (m, 2H), 3.27 - 3.22 (m, 2H). ¹³C NMR (101 MHz, CDCl₃) δ 69.7, 66.7, 50.6, 50.4, 39.7.

### 2-(2-(2-Azidoethoxy)ethoxy)ethan-1-amine (384)

¹H NMR (400 MHz, CDCl₃) *δ* 3.65 - 3.54 (m, 8H), 3.48 (t, *J* = 5.2 Hz, 2H), 3.37 - 3.32 (m, 2H). ¹³C NMR (101 MHz, CDCl₃) δ 72.7, 70.6, 70.2, 70.0, 50.6, 41.4.

### 17-Azido-3,6,9,12,15-pentaoxaheptadecan-1-amine (397)

¹H NMR (400 MHz, CDCl₃) *δ* 3.73 - 3.68 (m, 2H), 3.67 - 3.57 (m, 18H), 3.42 - 3.32 (m, 2H), 3.12 - 3.05 (m, 2H). ¹³C NMR (101 MHz, CDCl₃) *δ* 70.5, 70.4, 70.4, 70.2, 70.2, 70.1, 70.0, 69.9, 67.6, 50.6, 39.6, 22.9.

### Preparation of intermediates 412, 385, 363 and 416

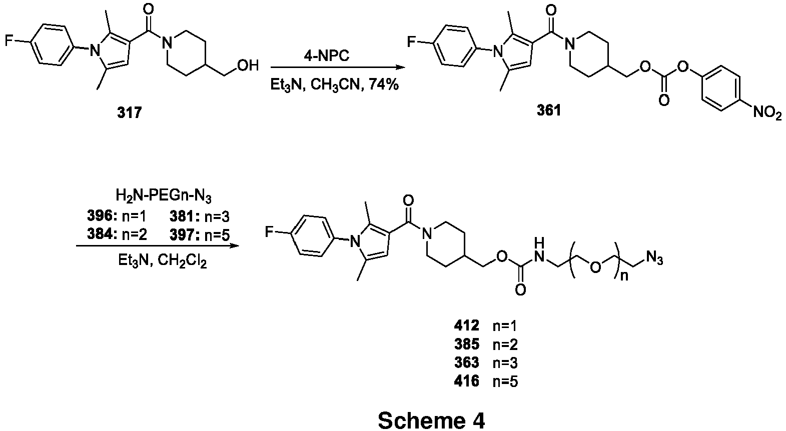

### [1-[1-(4-fluorophenyl)-2,5-dimethyl-1H-pyrrole-3-carbonyl]piperidin-4-yl]methyl (4-nitrophenyl) carbonate (361)

To a stirred solution of alcohol **317** (307 mg, 0.93 mmol) in 10 mL of anhydrous CH₂Cl₂, pyridine (224 µL, 2.79 mmol) and p-nitrophenyl chloroformate (468 mg, 2.32 mmol) were sequentially added. After stirring at rt for 30 min, the mixture was quenched with saturated aqueous NH₄Cl (10 mL) and extracted with CH₂Cl₂ (3 x 10 mL). The combined organic layers were dried over MgSO₄, filtered and concentrated in vacuo. The residue was purified by silica gel flash column chromatography (from 50% to 60% EtOAc in hexanes) to yield carbonate **361** (342 mg, 74% yield).

¹H NMR (400 MHz, CDCl₃) *δ* 8.34-8.25 (m, 2H), 7.44-7.35 (m, 2H), 7.22-7.13 (m, 4H), 5.97 (s, 1H), 4.54 (br s, 2H), 4.20 (d, 2H), 2.92 (br s, 2H), 2.13-2.03 (m, 1H), 2.09 (s, 3H), 1.98 (s, 3H), 1.89-1.83 (m, 2H), 1.36 (qd, *J* = 12.4, 4.3 Hz, 1H).¹³C NMR (101 MHz, CDCl₃) *δ* 171.1, 167.9, 163.4, 160.9, 155.5, 152.5, 145.4, 134.1, 130.9, 130.0, 129.9, 128.2, 125.3, 121.8, 116.4, 116.1, 114.8, 106.3, 73.1, 60.4, 35.9, 21.0, 14.2, 12.6, 12.0.

HRMS calculated for C₂₆H₂₇FN₃O₆⁺ [M+H]⁺: 496.1878; found: 496.1879.

### [(1-(1-(4-Fluorophenyl)-2,5-dimethyl-1H-pyrrole-3-carbonyl)piperidin-4-yl)methyl (2-(2-azidoethoxy)ethyl)carbamate] (412)

A solution of **361** (150 mg, 0.303 mmol) in dry CH₂Cl₂ (0.5 mL) was added dropwise to a stirred solution of N₃-PEG(1)-NH₂ **396** (59 mg, 0.454 mmol) and TEA (126 µL, 0.908 mmol) in dry CH₂Cl₂ (0.6 mL) at rt. After overnight stirring, the reaction mixture was concentrated in vacuo, and the resulting crude was purified by silica gel flash column chromatography to furnish carbamate **412** (100 mg, 68% yield).

¹H NMR (400 MHz, CDCl₃) *δ* 7.15 - 7.11 (m, 4H), 5.92 (s, 1H), 3.94 (d, *J* = 6.2 Hz, 6H), 3.62 (t, *J* = 5.3 Hz, 2H), 3.54 (t, *J* = 5.1 Hz, 7H), 3.38 -3.32 (m, 4H), 2.03 (s, 3H), 1.93 (s, 3H), 1.93 - 1.85 (m, 1H), 1.75 (d, *J* = 12.6 Hz, 2H), 1.30 - 1.17 (m, 3H).

¹³C NMR (101 MHz, CDCl₃) *δ* 168.8, 163.7, 161.0, 156.9, 133.8, 131.3, 129.9, 129.8, 128.8, 116.5, 116.3, 114.1, 106.2, 70.1, 70.0, 68.8, 53.5, 50.7, 40.9, 36.1, 12.7, 12.1. HRMS calcd for C₂₄H₃₂FN₆O₄⁺([M+H]⁺): 487.2469, found: 487.2387.

### [(1-(1-(4-fluorophenyl)-2,5-dimethyl-1H-pyrrole-3-carbonyl)piperidin-4-yl)methyl (2-(2-(2-azidoethoxy)ethoxy)ethyl)carbamate] (385).

Following an analogous procedure to that described for 412 but using N₃-PEG(2)-NH₂ **384,** intermediate **385** was obtained (173 mg, 81% yield).

¹H NMR (400 MHz, CDCl₃) *δ* 7.21 - 7.15 (m, 4H), 5.97 (s, 1H), 5.25 - 5.19 (m, 1H), 3.98 (d, 2H), 3.72 - 3.69 (m, 2H), 3.68 - 3.64 (m, 4H), 3.59 (t, 2H), 3.46 - 3.36 (m, 4H), 2.08 (s, 3H), 1.98 (s, 3H), 1.96 - 1.88 (m, 1H), 1.84 - 1.67 (m, 4H), 1.37 - 1.22 (m, 2H). ¹³C NMR (101 MHz, CDCl₃) *δ* 168.0, 163.4, 160.9, 156.7, 134.1, 130.7, 130.0, 129.9, 128.2, 116.4, 116.1, 114.9, 106.3, 70.6, 70.3, 70.1, 70.1, 68.8, 50.7, 40.8, 36.3, 29.2, 12.7, 12.0.

HRMS calcd for C₂₆H₃₆FN₆O₅⁺ ([M+H]⁺): 531.2731, found: 531.2859.

### 1-[1-(4-Fluorophenyl)-2,5-dimethyl-1H-pyrrole-3-carbonyl]piperidin-4-yl]methyl [2-[2-[2-(2-azidoethoxy)ethoxy]ethoxy]ethyl]carbamate (363)

Following an analogous procedure to that described for 412 but using N₃-PEG(3)-NH₂ **381,** intermediate **363** was obtained (238 mg, 82% yield).

¹H NMR (400 MHz, CDCl₃) *δ* 7.21-7.10 (m, 4H), 5.93 (s, 1H), 4.45 (br s, 2H), 3.95 (s, 2H), 3.70-3.61 (m, 11H), 3.56-3.51 (m, 2H), 3.41-3.33 (s, 2H), 2.86 (br s, 2H), 2.05 (d, 4H), 1.95 (d, 5H), 1.89 (d, 1H), 1.76 (d, 4H), 1.25 (dt, 3H). ¹³C NMR (101 MHz, CDCl₃) *δ* 167.9, 163.4, 161.0, 156.8, 134.2, 130.7, 130.0, 130.0, 128.2, 116.4, 116.2, 115.0, 106.3, 77.5, 77.2, 76.8, 70.8, 70.7, 70.3, 70.1, 68.8, 53.5, 50.7, 40.9, 36.4, 29.3, 12.7, 12.0.

HRMS calcd for C₂₈H₄₀FN₆O₆⁺ ([M+H]⁺): 575.2988, found: 575.2983.

### [(1-(1-(4-Fluorophenyl)-2,5-dimethyl-1H-pyrrole-3-carbonyl)piperidin-4-yl)methyl (17-azido-3,6,9,12,15-pentaoxaheptadecyl) carbamate] (416)

Following an analogous procedure to that described for 412 but using N₃-PEG(5)-NH₂ **397,** intermediate **416** was obtained (166 mg, 83% yield).

¹H NMR (400 MHz, CDCl₃) 7.20 - 7.13 (m, 4H), 5.95 (s, 1H), 5.38 - 5.32 (m, 1H), 3.96 (d, 2H), 3.68 - 3.64 (m, 14H), 3.64 - 3.60 (m, 4H), 3.56 (t, 2H), 3.42 - 3.33 (m, 4H), 2.07 (s, 3H), 1.96 (s, 3H), 1.95 - 1.87 (m, 1H), 1.83 - 1.74 (m, 2H), 1.34 - 1.20 (m, 3H). ¹³C NMR (101 MHz, CDCl₃)*δ* 167.9, 163.4, 160.9, 156.7, 134.1, 130.7, 130.0, 129.9, 128.1, 116.3, 116.1, 114.9, 106.3, 70.7, 70.7, 70.6, 70.6, 70.5, 70.3, 70.1, 70.0, 68.8, 53.4, 50.7, 40.8, 36.3, 29.2, 12.6, 12.0.

HRMS calcd for C₃₂H₄₈FN₆O₈⁺([M+H]⁺): 663.3517, found: 663.3559.

### B. Synthesis of Compounds of Formula I

### Preparation of RBM3-413, RBM3-386, RBM3-368 and RBM3-419

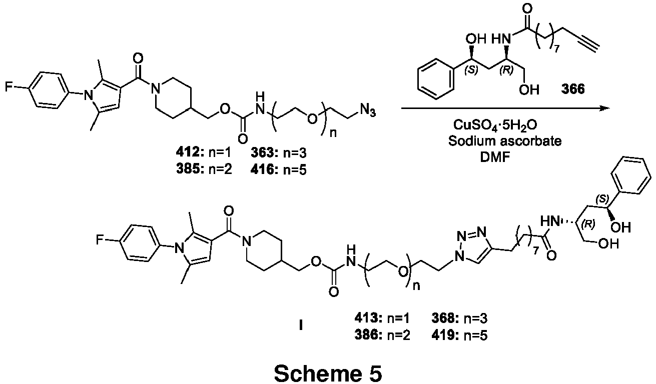

### [(1-(1-(4-Fluorophenyl)-2,5-dimethyl-1H-pyrrole-3-carbonyl)piperidin-4-yl)methyl (2-(2-(4-(9-(((2R,4S)-1,4-dihydroxy-4-phenylbutan-2-yl)amino)-9-oxononyl)-1H-1,2,3-triazol-1-yl)ethoxy)ethyl)carbamate] (RBM3-413).

**412** (60 mg, 0.123 mmol) and **366** (44 mg, 0.128 mmol) were solubilized in a round bottom flask with DMF (6 mL). This step was immediately followed by the addition of an aqueous solution of CuSO₄ (370 µL, 1 M) and an aqueous solution of sodium ascorbate (185 µL, 1 M). The resulting mixture was stirred at rt for 10 min and then concentrated and purified by flash column chromatography (from 0 to 10% MeOH in CH₂Cl₂) to obtain **RBM3-413** (85 mg, 83% yield) as a cream-colored wax.

¹H NMR (400 MHz, CDCl₃) *δ* 7.28 - 7.21 (m, 4H), 7.17 - 7.12 (m, 1H), 7.10 - 7.02 (m, 4H), 6.90 - 6.80 (m, 1H), 5.85 (s, 1H), 5.08 (br s, 1H), 4.72 (d, 1H), 4.54 - 4.25 (m, 4H), 4.03 - 3.97 (m, 1H), 3.92 - 3.83 (m, 2H), 3.81 - 3.73 (m, 2H), 3.65 - 3.55 (m, 2H), 3.46 - 3.36 (m, 2H), 3.27 - 3.21 (m, 2H), 2.10 (t, 3H), 2.00 - 1,94 (m, 1H), 1.97 (s, 3H), 1.90 - 1.80 (m, 1H), 1.88 (s, 3H), 1.73 - 1.63 (m, 3H), 1.509- 1.51 (m, 2H), 1.34 - 1.11 (m, 11H). ¹³C NMR (101 MHz, CDCl₃) *δ* 163.4, 160.9, 156.6, 144.8, 133.9, 130.8, 130.0, 129.9, 128.4, 127.3, 125.6, 116.4, 116.2, 106.2, 71.5, 70.1, 68.8, 65.4, 53.5, 50.5, 41.2, 40.6, 36.7, 3623, 28.1, 25.6, 12.7, 12.0.

HRMS calcd for C₄₅H₆₃FN₇O₇⁺ ([M+H]⁺): 832.4773, found: 832.4810.

### [(1-(1-(4-Fluorophenyl)-2,5-dimethyl-1H-pyrrole-3-carbonyl)piperidin-4-yl)methyl (2-(2-(2-(4-(9-(((2R,4S)-1,4-dihydroxy-4-phenyl butan-2-yl)amino)-9-oxononyl)-1H-1,2,3-triazol-1-yl)ethoxy)ethoxy)ethyl) carbamate] (RBM3-386).

Following an analogous procedure to that described for RBM3-413, but using **385** (50 mg, 0.094 mmol), **RBM3-386** (37 mg, 44% yield) was obtained as a cream-colored wax.

¹H NMR (400 MHz, MeOD) δ 7.28 - 7.09 (m, 10H), 5.87 (s, 1H), 4.59 (t, 1H), 4.45 (t, 2H), 3.86 - 3.71 (m, 4H), 3.76 - 3.65 (m, 1H), 3.51 - 3.43 (m, 4H), 3.42 - 3.34 (m, 4H), 3.15 (t, 2H), 2.15 - 2.05 (m, 2H), 1.92 (s, 3H), 1.88 (s, 3H), 1.86 - 1.77 (m, 1H), 1.72 - 1.60 (m, 4H), 1.56 - 1.48 (m, 3H), 1.38 - 1.11 (m, 12H).¹³C NMR (101 MHz, MeOD) δ 144.6, 130.7, 130.1, 130.0, 128.4, 128.0, 127.0, 125.9, 116.1, 115.8, 105.7, 71.2, 70.0, 69.9, 69.6, 68.8, 68.3, 63.4, 53.4, 40.3, 40.0, 36.0, 35.8, 29.0, 28.9, 28.9, 28.8, 25.6, 11.3, 10.7.

HRMS calcd for C₄₇H₆₇FN₇O₈⁺ ([M+H]⁺): 876.5035, found: 876.5092.

### [1-(1-(4-Fluorophenyl)-2,5-dimethyl-1H-pyrrole-3-carbonyl)piperidin-4-yl)methyl (2-(2-(2-(2-(4-(9-(((2R,4S)-1,4-dihydroxy-4-phenylbutan-2-yl)amino)-9-oxononyl)-1H-1,2,3-triazol-1-yl)ethoxy)ethoxy)ethoxy)ethyl)carbamate] (RBM3-368).

Following an analogous procedure to that described for RBM3-413, but using **363** (25 mg, 44 µmol), **RBM3-368** (31 mg, 77%) was obtained as a cream-colored wax.

¹H NMR (400 MHz, CDCl₃) *δ* 7.47 (s, 1H), 7.36-7.28 (m, 4H), 7.26-7.20 (m, 1H), 7.16 (m, 4H), 6.80 (d, 1H), 5.93 (s, 1H), 5.42-5.33 (m, 1H), 4.82 (dd, 1H), 4.48 (t2H), 4.12-4.03 (m, 1H), 3.94 (br d, 2H), 3.85 (t, 2H), 3.67 (d, 2H), 3.61-3.56 (m, 9H), 3.54 (t, 2H), 3.38-3.32 (m, 2H), 2.67 (t, 2H), 2.18 (t, 2H), 2.09-2.06 (m, 1H), 2.05 (s, 3H), 1.96 (s, 3H), 1.95-1.88 (m, 2H), 1.80-1.71 (m, 2H), 1.69-1.57 (m, 4H), 1.36-1.19 (m, 11H). ¹³C NMR (101 MHz, CDCl₃) *δ* 144.8, 134.2, 130.7, 130.1, 130.0, 128.5, 128.4, 127.5, 125.8, 116.5, 116.3, 106.3, 71.8, 70.6, 70.6, 70.4, 70.2, 68.9, 41.3, 40.9, 36.9, 36.4, 29.4, 29.0, 25.7, 12.8, 12.1.

HRMS calcd for C₄₉H₇₁FN₇O₉⁺ ([M+H]⁺): 920.5292, found: 920.5295.

### [(1-(1-(4-Fluorophenyl)-2,5-dimethyl-1H-pyrrole-3-carbonyl)piperidin-4-yl)methyl (17-(4-(9-(((2R,4S)-1,4-dihydroxy-4-phenylbutan-2-yl)amino)-9-oxononyl)-1H-1,2,3-triazol-1-yl)-3,6,9,12,15-pentaoxaheptadecyl)carbamate] (RBM3-419)

Following an analogous procedure to that described for RBM3-413, but using **416** (65 mg, 0.098 mmol), **RBM3-419** (40 mg, 40% yield) was obtained as a cream-colored wax.

¹H NMR (400 MHz, MeOD) *δ* 7.41 - 7.21 (m, 10H), 5.99 (s, 1H), 4.71 (t, 1H), 4.59 - 4.55 (m, 2H), 3.96 (d, 2H), 3.93 - 3.88 (m, 2H), 3.87 - 3.79 (m, 1H), 3.69 - 3.59 (m, 17H), 3.5 6-3.46 (m, 3H), 3.29 (t, 2H), 2.21 (t, 2H), 2.04 (s, 3H), 2.00 (s, 3H), 2.02 - 1.91 (m, 2H), 1.84 - 1.76 (br s, 2H), 1.73 (br s, 2H), 1.64 (br s, 2H), 1.43 - 1.24 (m, 12H). ¹³C NMR (101 MHz, CDCl₃) *δ* 144.6, 134.1, 130.7, 130.1, 130.0, 128.4, 128.0, 127.0, 125.9, 116.1, 115.8, 105.7, 71.2, 70.2, 70.2, 70.1, 69.9, 69.6, 68.9, 68.3, 63.4, 53.4, 40.3, 40.0, 36.0, 35.8, 29.0, 28.9, 28.9, 28.8, 25.6, 11.3, 10.7.

HRMS calcd for C₅₃H₇₉FN₇O₁₁⁺ ([M+H]⁺): 1008.5822, found: 1008.5834.

### Preparation of RBM3-395

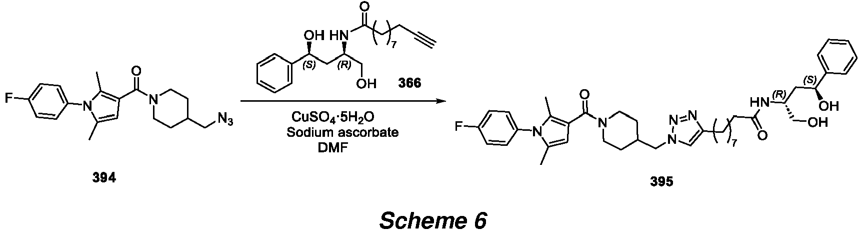

### [N-((2R,4S)-1,4-Dihydroxy-4-phenylbutan-2-yl)-9-(1-((1-(1-(4-fluorophenyl)-2,5-dimethyl-1H-pyrrole-3-carbonyl)piperidin-4-yl)methyl)-1H-1,2,3-triazol-4-yl)nonanamide] (RMB3-395).

**394** (25 mg, 0.070 mmol) and **366** (25.3 mg, 0.073 mmol) were solubilized in a round bottom flask with DMF (3.5 mL). This step was immediately followed by the addition of an aqueous solution of CuSO₄ (125 µL, 1 M) and an aqueous solution of sodium ascorbate (106 µL, 1 M). The resulting mixture was stirred at rt for 10 min and then concentrated and purified by flash column chromatography (from 0 to 10% MeOH in CH₂Cl₂) to obtain **RBM3-395** (36 mg, 73%) as a cream-colored wax.

¹H NMR (400 MHz, CDCl₃) *δ* 7.33 - 7.26 (m, 5H), 7.25 - 7.19 (m, 1H), 7.17 - 7.10 (m, 4H), 6.85 (d, 1H), 5.90 (s, 1H), 4.80 (dd, 1H), 4.44 (br s, 2H), 4.24 - 4.15 (m, 3H), 4.09 - 4.02 (m, 1H), 3.65 (d, 2H), 2.91 - 2.76 (br s, 2H), 2.68 (t, 2H), 2.16 (t, 3H), 2.06 - 2.02 (m, 1H), 2.02 (s, 3H), 1.94 (s, 3H), 1.93 - 1.87 (m, 1H), 1.68 - 1.57 (m, 6H), 1.31 - 1.19 (m, 11H). ¹³C NMR (101 MHz, CDCl₃) *δ* 168.29, 161.08, 144.78, 134.00, 131.19, 130.05, 129.97, 128.52, 127.49, 125.74, 116.54, 116.32, 114.48, 106.28, 71.70, 65.62, 50.65, 41.19, 36.69, 28.95, 28.66, 25.66, 12.75, 12.09.

HRMS calcd for C₄₀H₅₄FN₆O₄⁺ ([M+H]⁺): 701.4191, found: 701.4178.

### Cell Biology Methods.

### Cell cultures and incubations with compounds.

MDA-MB-231 cells were grown in DMEM media (Sigma) supplemented with 10% fetal bovine serum (FBS) (Sigma), 2 mM L-glutamine (Thermo Fisher) and 100 U/mL penicillin/ 100 ug/mL streptomycin (Thermo Fisher). MDA-MB-453 cells were grown in DMEM/F12 media (Gibco) supplemented with 10% fetal bovine serum (FBS) (Sigma), 2 mM L-glutamine (Thermo Fisher), 2.5 ug/mL amphotericin B and 100 U/mL penicillin/ 100 ug/mL streptomycin (Thermo Fisher). BT-474 cells were grown in DMEM/F12 media (Gibco) supplemented with 10% fetal bovine serum (FBS) (Sigma), 2 mM L-glutamine (Thermo Fisher), 10 ug/mL insulin, 2.5 ug/mL amphotericin B and 100 U/mL penicillin/ 100 ug/mL streptomycin (Thermo Fisher).

Cells were incubated with Protacs or DMSO (control) for the times and concentrations indicated in the figures. Then, medium was removed and cells were washed with PBS, trypsinized and the pellets were disrupted by probe sonication in a 0.25 M solution of Saccharose. An aliquot was used for protein quantification and the same amount of protein per treatment was treated with laemli buffer (95 ºC/5 min). The protein extracts were analyzed by SDS-PAGE electrophoresis followed by immunoblot using the following antibodies: CERT1, Abcam Ab72536; USP14, Cell signaling CS-11931; Actin, Abcam Ab5694.

### Cell viability assays in human cells.

For cell proliferation assays, cells were plated at a density of 10,000 cells/well in a 96-well plate. Upon 16 h of culture, cells were treated with increasing doses of compounds, and cultured for 24h. MTT reagent was added to cell culture media. After 3 h incubation, culture medium was replaced by DMSO. MTT formazan crystals were solubilized in DMSO using a plate agitator for 30 min prior to reading absorbance at 570 nm with a SYNERGY H1 microplate reader (BioTek

### Degradation of CERT in cultured human breast cancer cells

Chimeras were tested in different types of breast cancer cell lines:
- MDA-MB-231, lacking the estrogen receptor (ER), progesterone receptor (PR) and human epidermal growth factor receptor-2 (HER2). This triple negative breast cancer cell line was used by Swanton et al. (Cancer Cell. 2007;11(6):498-512) where they showed that silencing of CERT correlated with lower proliferation and higher sensitivity to chemotherapic drugs (i. e. paclitaxel, doxorubicin), which suggested that CERT could be a therapeutic target in oncology.
- MDA-MB-453, lacking estrogen receptor (ER) and progesterone receptor (PR), and expressing HER2. These cells may develop resistance to HER2-targeted therapies, such as lapatinib or trastuzumab treatments.
- BT-474, human ductal breast carcinoma cell line, expressing ER, PR and HER2, which may exhibit resistance to HER2-tageted treatments, as well.

Compounds RBM3-413, RBM3-395, RBM3-386, RBM3-368 and RBM3-419 induced the degradation of CERT in MDA-MB-231 (Figure 1A), MDA-MB-453 (Figure 1B) and BT-474 (Figure 1C). Cells incubated for 24 h in the presence of 10 µM of each compounds exhibited a decrease in CERT levels.

MDA-MB-231 cells treated with RBM3-413, RBM3-395, RBM3-386, RBM3-368 and RBM3-419 in the 1-40 µM range (including concentrations at 1, 5, 10, 20, 30 and 40 µM) for 24 h, exhibited distinct dose-responses (Figures 2A to 2E).

Wild type and lapatinib-resistant MDA-MB-453 cells treated with RBM3-413, RBM3-395, RBM3-386, RBM3-368 and RBM3-419 in the 1-40 µM range (including concentrations at 1, 5, 10, 20, 30 and 40 µM) for 24 h, exhibited distinct dose-responses (Figure 3, where panels A -E, show treatments of MDA-MB-453/WT cells, and Figure 4, panels F-J represent treatments of MDA-MB-453 lapatinib-resistant cells with indicated compounds).

Wild type and lapatinib-resistant BT-474 cells treated with RBM3-413, RBM3-395, RBM3-386, RBM3-368 and RBM3-419 in the 1-40 µM range (including concentrations at 1, 5, 10, 20 and 40 µM) for 24 h, exhibited distinct dose-responses (Figure 5, where panels A-E show treatments of BTB-474/WT cells, and Figure 6, panels F-J represent treatments of BTB-474 Lapatinib-resistant cells with indicated compounds).

Overall, the maximum degradation response is observed within the 10-40 µM range.

Time course assays were performed with RBM3-368 in MDA-MB-231 cells, by means of incubations for 2 h, 4 h, 6 h, 8 h, 16 h and 24 h. These assays showed CERT degradation effect in 16-24 h, with a maximum degradation observed at 24 h (Figure 7).

### Sensitization of cancer cells to lapatinib treatments

Viability of MDA-MB-453 cells, both WT and lapatinib (Lap) resistant, treated with lapatinib (10 µM) in the absence or the presence of the **RBM3-395** or **RBM3-413** (10 µM) was measured, as shown in Figure 8. Lapatinib treatment reduced the viability of MDA-MB-453/WT cells (-78%), but not that of MDA-MB-453/Lap cells. The presence of **RBM3-395** and **RBM3-413** caused a strong decrease in viability (-38%) in both MDA-MB-453/WT and MDA-MB-453/Lap cells.

Lapatinib dose-response assays in MDA-MB-453/WT and MDA-MB-453/Lap cells, in the absence or presence of **RBM3-413, RBM3-395, RBM3-386, RBM3-368** and **RBM3-419** (10 µM) (Figure 9, where panels A-C and Figure 10, where panels D-E, show treatments of MDA-MB-453/WT cells, and Figure 10, panel F and Figure 11, panels G-J represent treatments of MDA-MB-453 lapatinib-resistant cells with indicated compounds). In MDA-MB-453/Lap cells, both **RBM3-395** and **RBM3-413** caused a potent sensitization to Lapatinib treatments, as concluded from the shift of their dose response curves towards the left as compared to lapatinib in vehicle only (Figure 10F and 11G, respectively). Sensitization was also observed, yet mildly, in MDA-MB-453/WT cells, in the presence of **RBM3-395** or **RBM3-413** (Figure 9A and 9B). Thus, the CC50 vehicle /CC50 RBM3-413 ratios exhibited values of 1.98 in WT cells and 2.33 in lapatinib resistant cells, while the CC50 vehicle /CC50 RBM3-395 ratios exhibited values of 2.27 in WT cells and 2.05 in lapatinib resistant cells. Statistical analysis of the lapatinib dose-response curves with or without RBM3-413 or RBM3-395 indicate that the difference in the CC50 values is statistically significant (P<0,0001).

Lapatinib dose-response assays in BT-474/WT and BT-474/Lap cells, in the absence or presence of the indicated Protacs (20 µM) showed that only those with the shorter linker **(RBM3-395** and **RBM3-413)** sensitized cells to lapatinib treatments (Figure 12). As shown in Figures 14G-J, both **RBM3-395** and **RBM3-413** caused a potent sensitization to Lapatinib treatments, as concluded from the shift of their dose response curves towards the left as compared to lapatinib in vehicle only. Thus, the CC50 vehicle /CC50 RBM3-413 ratios exhibited values of 3.84 in WT cells and 5.56 in lapatinib resistant cells, while the CC50 vehicle /CC50 RBM3-395 ratios exhibited values of 6.22 in WT cells and 5.23 in lapatinib resistant cells. Statistical analysis of the lapatinib dose-response curves with or without RBM3-413 or RBM3-395 indicate that the difference in the CC50 values is statistically significant (P<0,0001).

## Claims

1. A compound of formula **I**: 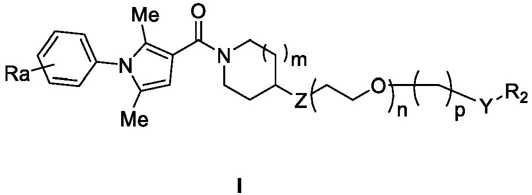
or a pharmaceutical salt thereof, wherein
Rₐ represents -H, -F, -Cl, -Br, -I, -CN, -(C₁-C₄)-alkyl, OR_{b}, -NHR_{b}R_{c}, or NO₂;
-m is an integer number between 0 and 1;
Z represents -CH₂-, -CH₂OCONR_{d}-, -CH₂NRₑCONR_{d}-, -CH₂NRₑCO- or -CH₂CONR_{d}-;
Each R_{b}, R_{c}, R_{d} and Rₑ independently represents -H- or -(C₁-C₄)-alkyl;
n is an integer number between 0 and 10;
p is an integer number between 0 and 3;
Y represents (CH₂)r or a group of formula **II**: 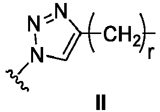
r is an integer number between 6 and 12;
R₂ represents a group of formula **III** or **IV:** 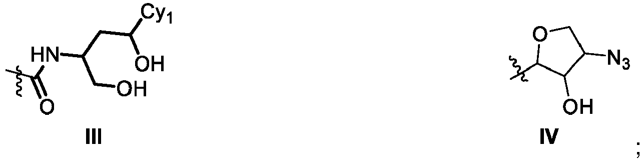 and
Cy, represents an aromatic carbocycle group from 5 to 10 carbon atoms.

2. The compound of formula **I** according to claim 1, wherein Rₐ is selected from - F, -Cl and -Br.

3. The compound of formula **I** according to claim 1 or 2, wherein m is 1.

4. The compound of formula **I** according to claim 1 or 3, wherein Z is -CH₂ or - CH₂OCONR_{d}-.

5. The compound of formula **I** according to claim 1 or 4, wherein n is an integer number from 0 to 5.

6. The compound of formula I according to claim 1 or 5, wherein p is an integer number from 0 to 2.

7. The compound of formula I according to any od claims 1 to 6, wherein r is 7.

8. The compound of formula I according to claim 1 or 7, wherein R₂ is a group of formula **III.**

9. The compound of formula **I** according to claim 1, which is selected from:

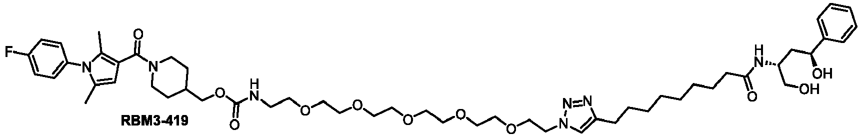

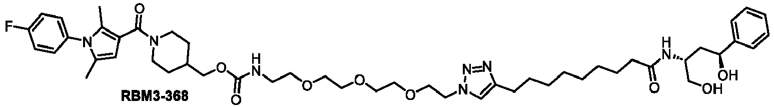

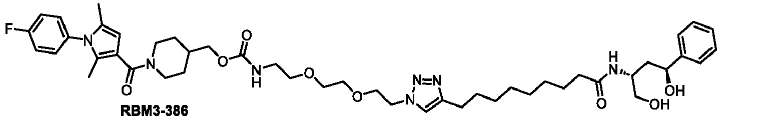

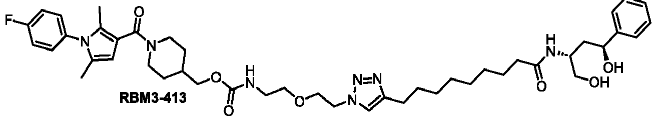

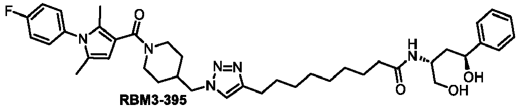

10. A pharmaceutical composition, which comprises a compound of formula **I** according to any of claims 1 to 9 or a pharmaceutically acceptable salt thereof and one or more pharmaceutically acceptable excipients.

11. A compound of formula **I** according to any of claims 1 to 10 or a pharmaceutical salt thereof for use as a medicament.

12. A compound of formula **I** according to any of claims 1 to 10 or a pharmaceutical salt thereof for use in the treatment of a cancer related to CERT.

13. The compound of formula **I** or a pharmaceutical salt thereof for the use according to claim 12, wherein the cancer related to CERT is selected from lung, breast, colon, liver and ovary.
